# EUROPEAN PATENT APPLICATION

(11) **EP 3 505 819 A1**
(43) Date of publication of application: **03.07.2019**
(21) Application number: 17860516.8
(22) Date of filing: 12.10.2017
(51) Int. Cl.: F21V 8/00

(54) **MEDICAL LIGHT SOURCE MODULE AND MEDICAL LIGHT SOURCE DEVICE INCLUDING SAME**

(30) Priority: 13.10.2016 KR 20160133163
(71) Applicant: Korea Electro Technology Research Institute, Changwon-si, Gyeongsangnam-do 51543 (KR); Inthesmart Co. Ltd., Seoul 03082 (KR)
(72) Inventor: BAE, Soo Jin, Seoul 07519 (KR); LEE, Dae Sic, Seoul 03915 (KR); KIM, Han Suk, Seoul 07055 (KR); JUNG, Min Woong, Seoul 02050 (KR)
(74) Representative: ABG Intellectual Property, S.L.
(86) International application number: PCT/KR2017/011268
(87) International publication number: WO 2018/070813

(57) **Abstract**

The present invention relates to a medical light source, and more particularly, to a medical light source module transmitting light to an optical fiber of an endoscope and a medical light source device including the same. The present invention discloses a medical light source module including: an optical system part in which an optical fiber is coupled to one side, and a first coupling surface (101), which is perpendicular to an optical axis (L), is provided at the other side with respect to the optical axis (L), and to which at least one lens is mounted to provide an optical system; and a light source part (500) having a second coupling surface (102), which surface-contacts the first coupling surface (101), and installed so that an optical axis of a light source is perpendicular to the second coupling surface (102).

## Description

### [TECHNICAL FIELD]

The present invention disclosed herein relates to a medical light source, and more particularly, to a medical light source module transmitting light to an optical fiber of a medical device such as an endoscope and a laparoscope and a medical light source device including same.

### [BACKGROUND ART]

In general, an endoscope has widely used for a surgery or a health examination of a patient in hospitals, and the endoscope necessarily includes a light source as a lighting for observing and photographing an object.

The endoscope photographs a narrow space such as the inside of a human body or the inside of a machine as an image in order to observe the narrow space. In particular, the endoscope in the medical field allows to observe the inside of the human body (stomachs, bronchial tubes, esophagi, large intestines, small intestines, etc.) to check whether it is normal by using a miniature camera without performing laparotomy or incision of the human body such as a surgery or an autopsy.

Currently, the endoscope is used for various industrial fields including the medical field, e.g., observation of the inside of a precision machine without disassembly or observation to check whether the inside of a pipe is normal.

In a generally well-known conventional endoscope system, a camera is disposed on a front end of the endoscope. The camera includes: a lighting unit emitting light to watch a human internal organ or an inner surface of a machine; an imaging element receiving an optical signal, which is obtained such that light emitted from the lighting unit is incident to and reflected from a surface of a human internal organ, to convert the optical signal into an electrical signal (image signal); and a camera chip including an encoder for converting the image signal into an electronic signal to observe through a monitor.

Also, the lighting unit, which is an electrical light emitting unit, uses a lamp or LED as a light source, and the light source is directly installed on the front end of the endoscope or allows the light transmitted through the optical fiber to be illuminated.

The light source device, which is used for medical equipment such as the endoscope, is disclosed in Korean Registered Patent No. 10-1657887. The light source device used for the medical equipment such as the endoscope includes an optical system which is a high power light source allowing the light to be efficiently transmitted to an observation part by focusing light of a high power LED in order to secure a view of the observation part such as the camera.

Here, when a component is necessary to be replaced because of, e.g., degradation of the LED, the component including the LED is required to be easily and quickly replaced in an emergency room or a medical office.

However, when the LED is replaced, precise coupling between the LED and the corresponding optical system, e.g., optical axis alignment, is necessary to efficiently transmit the light until the observation part. Thus, the replacement of the LED is difficult and cumbersome.

### [DISCLOSURE OF THE INVENTION]

### [TECHNICAL PROBLEM]

The present invention provides a medical light source module, which is convenient in maintenance because assembly between a light source part and an optical system and optical axis alignment between the light source and the optical system are easily performed due to simple assembly, and a medical light source device including same.

### [TECHNICAL SOLUTION]

In accordance with an embodiment of the present invention, a medical light source module includes: an optical system part in which an optical fiber is coupled to one side, and a first coupling surface 101, which is perpendicular to an optical axis L, is provided at the other side with respect to the optical axis L, and to which at least one lens is mounted to provide an optical system; and a light source part 500 having a second coupling surface 102, which surface-contacts the first coupling surface 101, and installed so that an optical axis of a light source is perpendicular to the second coupling surface 102.

The light source part 500 may include: a metal plate 510 having the second coupling surface; the light source coupled to the metal plate 510 so that the optical axis is perpendicular to the second coupling surface 102; and a heat dissipation block 520 coupled to at least one of the metal plate 510 and the light source to dissipate heat generated from the light source.

The light source may include a LED device 281 and a LED board 282 on which the LED device 281 is installed and installed on the second coupling surface 102.

The optical system part may include: at least one lens 271 and 272 configured to guide light along the optical axis L; an optical fiber coupling part 290 installed opposite to the light source with respect to the lens 271 and 272 and coupled to an optical fiber; a lower optical system holder 300 on which the lens 271 and 272 and the optical fiber coupling part 290 are seated so that the light source, the lens 271 and 272, and the optical fiber coupling part 290 are sequentially arranged to provide the optical axis L; an upper optical system holder 400 disposed above the lower optical system holder 300 and coupled to the lower optical system holder 300 so that the lens 271 and 272 and the optical fiber coupling part 290 are fixedly arranged along the optical axis L. Here, the first coupling surface 101 may be provided on at least one of the lower optical system holder 300 and the upper optical system holder 400, and the light source part 500 may be coupled to at least one of the lower optical system holder 300 and the upper optical system holder 400 to slide in a direction perpendicular to the optical axis L in a state in which the second coupling surface 102 closely contacts the first coupling surface 101.

The optical system part may further include a fixing block 360 which is disposed opposite to the optical fiber coupling part 290 with respect to the lens 271 and 272 to fix the lens 272 with respect to the lower optical system holder 300 and the upper optical system holder 400 and in which a through-hole 361 is defined to transmit light generated from the light source.

The fixing block 360 may be made of a metallic material to discharge heat transferred from the light source, and at least one of a plurality of projections and ribs may be formed on an outer circumferential surface to maximize a heat dissipation effect.

The medical light source module may further include a linear guide part configured to guide a slide movement of the light source part 500 with respect to the optical system part 30 in a direction perpendicular to the optical axis L in a state in which the second coupling surface 102 closely contacts the first coupling surface 101.

The linear guide part may include one pair of slide members 541 coupled to at least one of the lower optical system holder 300 and the upper optical system holder 400, and configured to guide a linear slide movement of the metal plate 510 in a direction perpendicular to the optical axis L while the second coupling surface 102 of the metal plate 510 closely contacts the first coupling surface 101.

A block coupling surface 525 of the heat dissipation block 520, which is perpendicular to the optical axis L, may further protrude in a front direction than the rest portion 524 so that the metal plate 510 is coupled to the heat dissipation block 520 while being spaced apart from both sides of the heat dissipation block 520.

Each of the one pair of slide members 541 may be a plate member that is elongated along the slide movement direction, and each of portions 341 and 441 coupled with the slide member 541 may further protrude toward the heat dissipation block 520 to prevent interference with the metal plate 510 when the lower optical system holder 300 and the upper optical system holder 400, which are coupled with the one pair of slide members 541, move in a slide manner.

At least one of the metal plate 510 and the heat dissipation block 520 may be additionally coupled to at least one stopper 531 that restricts a movement of the light source part 500 with respect to the lower optical system holder 300 and the upper optical system holder 400 so that the LED device 281 coupled to the metal plate 510 is exactly positioned on the optical axis L.

In accordance with another embodiment of the present invention, a medical light source device includes: a housing 10 installed to expose an operation panel 11, which is operated by a user, to the outside; and a light source module 20 installed to expose an optical fiber coupling part 290, which is disposed at one side of the housing 10 and coupled with an optical fiber, to the outside of the housing 10. Here, the light source module 20 is attached to and detached from the housing 10 in a sliding manner in a direction perpendicular to the optical axis L, and is the same as the light source module 20 having the above-described configuration.

The medical light source device may further include at least one auxiliary light source module 30 that is additionally coupled in the direction perpendicular to the optical axis L to transmit light in combination with light generated from the LED device 281 or independently transmit auxiliary light to an optical fiber coupled to the optical fiber coupling part 290.

### [ADVANTAGEOUS EFFECTS]

The medical light source module and the medical light source device including same in accordance with the present invention includes: the optical system part to which at least one lens is mounted to form the optical system; and the light source part having the second coupling surface, which surface-contacts the first coupling surface perpendicular to the optical axis, and installed so that the optical axis of the light source is perpendicular to the second coupling surface. Thus, the assembly of the optical system and the optical axis alignment may be easily performed, and the assembly and productivity may be remarkably enhanced due to the simple structure.

In particular, when replacement of the light source is urgent in an emergency room, the light source may be replaced by an unskilled person such as a nurse without difficulty, and thus convenient in usage.

In accordance with a more specific embodiment, the medical light source module and the medical light source device including same in accordance with the present invention may fix at least one lens constituting the optical system by the upper optical system holder and the lower optical system holder, which are vertically separated from each other, and sliding-couple the light source such as the LED device in the direction perpendicular to the optical axis, which is formed by the lens and the LED device, thereby precisely and stably installing the lens and the light source without misalignment of the optical axis.

Furthermore, the medical light source module and the medical light source device including same in accordance with the present invention may replace the light source such as the LED device in a state in which the light source part slides to be exposed to the outside when maintenance including the replacement of the light source such as the LED device is required, to easily perform the replacement of the light source without difficulty in alignment of the optical axis, which is formed by the optical system and the light source.

In particular, in order to slide the light source part with respect to the upper optical system holder and the lower optical system holder, as the metal plate and the heat dissipation block, which are coupled to the light source, are provided, and the linear movement of the slide member coupled to the upper optical system holder and the lower optical system holder is guided in a state in which the second coupling surface of the metal plate surface contacts the first coupling surface, which is formed on at least one of the lower optical system holder and the upper optical system holder of the optical system, the structure of the light source module may be simplified, and the light source part may be slidably coupled to the upper optical system holder and the lower optical system holder.

As at least one lens is seated on the upper optical system holder and the lower optical system holder, and the lens is further firmly fixed by the coupling between the upper optical system holder and the lower optical system holder, the optical system may be assembled without the misalignment of the optical system, and then the optical axis may be aligned even when the assembly is operated by an unskilled person.

Also, since the medical light source module necessarily includes the heat dissipation block for dissipating the heat generated from the light source, as the heat dissipation block is detachably coupled to the metal plate to which the light source is coupled, various heat dissipation blocks may be used without being restricted in coupling conditions and installation conditions of the heat dissipation block.

In particular, since the heat dissipation block has a limitation in holding pressure of the jig due to the various shapes and fin structure thereof, the heat dissipation block has a difficulty in perforation.

However, in accordance with the present invention, the position of the light source may be exactly fixed through the metal plate, and the heat generated from the light source may be transferred to the heat dissipation plate through the metal plate.

Also, since the heat dissipation block and the metal plate are unnecessary to be coupled at an exact position, the perforation process of the heat dissipation block may not require a high precision degree, and thus the convenience in manufacturing may be enhanced.

### [BRIEF DESCRIPTION OF THE DRAWINGS]

FIG. 1 is a perspective view illustrating an embodiment of a medical light source device including a medical light source module in accordance with the present invention.
FIG. 2 is a plan view illustrating the medical light source device of FIG. 1.
FIG. 3 is a perspective view illustrating the medical light source module installed on the medical light source device of FIG. 1.
FIG. 4 is an exploded perspective view illustrating the medical light source module of FIG. 3.
FIG. 5 is a cross-sectional view taken along line IV-IV in FIG. 3.
FIG. 6 is a plan view taken along line VI-VI in FIG. 3, illustrating a state in which an upper optical system holder is removed.
FIG. 7 is a plan view illustrating a state in which an auxiliary light source module is coupled to the medical light source module of FIG. 3.

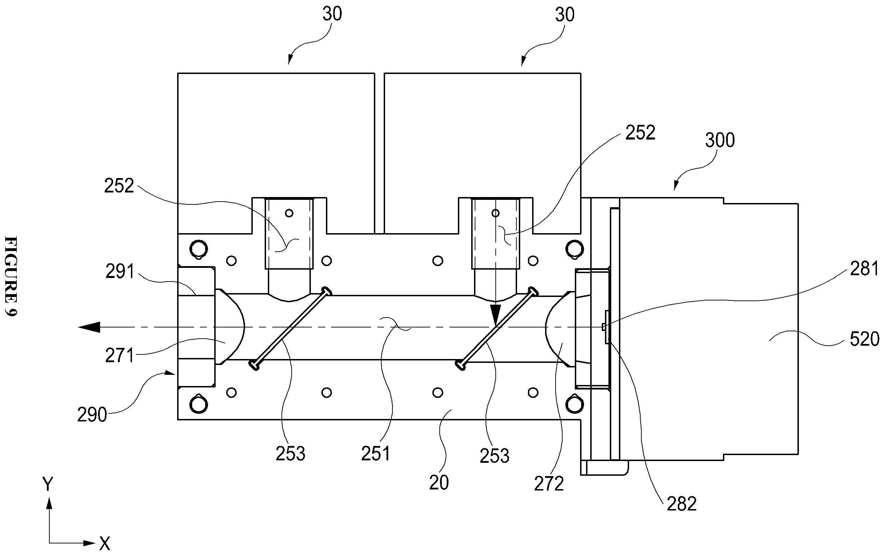

FIGS. 8 and 9 are plan views illustrating a state in which a plurality of auxiliary light source modules are coupled as a modified embodiment of FIG. 7.

### [MODE FOR CARRYING OUT THE INVENTION]

Hereinafter, a medical light source module and a medical light source device including same in accordance with the present invention will be described with reference to the accompanying drawings.

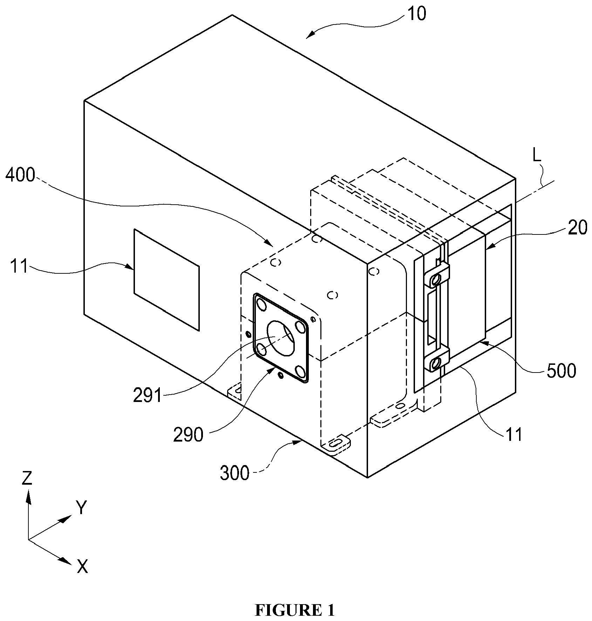

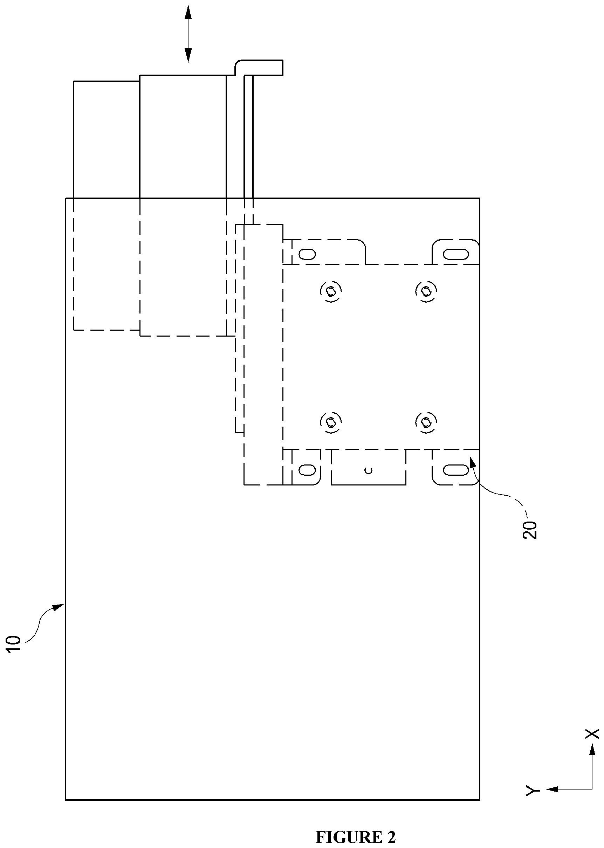

As illustrated in FIGS. 1 and 2, a medical light source device 10 in accordance with the present invention includes a housing 10 installed to expose an operation panel 11, which is operated by a user, to the outside and a light source module which is detachably coupled to the housing 10 and to which an optical fiber (not shown) is coupled.

As a component to which the light source module 20 is detachably coupled, the housing 10 may be variously provided according to a power supply structure and a coupling structure with the light source module 20.

For example, the housing 10 may accommodate, therein, a power supply unit (not shown) for supplying a power to the light source module 20 or the like and a control unit (not shown) for controlling the operation panel 11, the light source module 20, or the like.

As an operation panel installed to be exposed to the outside of the housing 10 for user's operation, the operation panel 11 may be variously provided according to operation manners.

As a component installed at one side of the housing 10 so that a portion thereof is exposed to the outside to be coupled with an optical fiber coupling adapter (not shown) coupled with an optical fiber, the light source module 20 may be variously provided.

Here, at least a component of the light source module 20, e.g., a light source part that will be described later, may be separated from the optical system that is the rest component, and the housing 10 may include an opening 12, which is able to be opened and closed by a door (not shown), in order to withdraw the at least a component of the light source module 20, e.g., the light source part that will be described later, to the outside.

Also, in accordance with a specific embodiment, the light source module 20 includes: an optical system part forming an optical system such that, with respect to an optical axis L, an optical fiber is coupled at one side, and a first coupling surface 101 perpendicular to the optical axis L is formed at the other side, and at least one lens is mounted; and a light source part 500 having a second coupling surface 102 that surface-to-surface contact the first coupling surface 101 and is installed so that an optical axis of the light source is perpendicular to the second coupling surface 102.

As a component having the second coupling surface 102, which surface-to-surface contacts the first coupling surface 101, and the optical axis of the light source, which is perpendicular to the second coupling surface 102, the light source part 500 may include any component including the light source and the second coupling surface 102.

Here, as the first coupling surface 101 and the second coupling surface 102 are perpendicular to the optical axis L while closely contacting each other, the optical axis of the light source coupled to the second coupling surface 102 while being aligned with the same may be aligned with the optical axis L of the optical system part without using an additional jig or member.

To this end, each of the first coupling surface 101 and the second coupling surface 102 may be processed or molded to have a flatness of a precision degree so that the first coupling surface 101 and the second coupling surface 102 are perpendicular to the optical axis L while closely contacting each other.

Also, the light source part 500 may include, e.g., a metal plate 510 having the second coupling surface 102, a light source coupled to the metal plate 510 so that the optical axis is perpendicular to the second coupling surface 102, and a heat dissipation block 520 coupled to at least one of light sources to dissipate heat generated from the light source.

The metal plate 510 may include any component having the second coupling surface 102 closely contacting the first coupling surface 101 of the optical system part that will be described later.

The metal plate 510 may be preferably made of a metallic material such as copper, and the second coupling surface 102 may be processed or molded to have a flatness of a precision degree, thereby being perpendicular to the optical axis L while closely contacting the first coupling surface 101.

Also, the metal plate 510 is coupled to the heat dissipation block 520 to discharge heat generated from the light source to the outside.

The heat dissipation block 520 may have various configurations and be selected according to a heat dissipation feature, a design, or the like.

The heat dissipation block 520 and the metal plate 510 may be coupled by a bolt or the like, and thermal grease may be applied therebetween.

In particular, as the light source part 500 includes the metal plate 510 and the heat dissipation block 520, which is separated from the metal plate 510, the heat dissipation block 520 may be freely changed or replaced.

Here, the metal plate 510 and the heat dissipation block 520 may be integrated with each other.

As a component dissipating the heat generated from the light source such as LED device 281, the heat dissipation block 520 is preferably made of a metallic material that has excellent heat dissipation characteristics.

The heat dissipation block 520 may include a plurality of fins to increase the heat dissipation characteristics and a fan 590 for providing air flow at surroundings of the heat dissipation block 520.

Also, a block coupling surface 525 of the heat dissipation block 520, which is perpendicular to the optical axis L, may protrude in a front direction further than the rest portion 524 so that the metal plate 510 is coupled to the heat dissipation block 520 while being spaced apart from both sides of the heat dissipation block 520.

That is, the heat dissipation block 520may have the block coupling surface 525, which is perpendicular to the optical axis L and protrudes in the front direction further than the rest portion 524, so that the metal plate 510 is coupled to the heat dissipation block 520 while being spaced apart from the both sides of the heat dissipation block 520.

As a component emitting light while being coupled to the metal plate 510 so that the optical axis is perpendicular to the second coupling surface 102, the light source may include various lighting units such as a LED device, a xenon lamp, a laser diode.

For example, the light source may include a LED device 281 and a LED board 282 on which the LED device 281 is installed and which is installed on the second coupling surface 102.

Also, the light source may be installed to allow the alignment of the optical axis L when coupled on the metal plate 510.

For example, the light source may be installed to allow the alignment of the optical axis L when coupled on the metal plate 510 to precisely adjust a coupling angle by using one or more bolts when coupled with the metal plate 510.

As a board on which the LED device 281 is installed, the LED board 282 may be variously provided according to an installation structure or a kind of the LED device 281 such as PCB.

Since the LED device 281 transmits light to the inside of the human body through an optical fiber according to a lighting purpose and a kind of a lighting, the LED device 281 preferably uses a high power LED device.

The optical system part is formed such that the optical fiber is coupled at one side and the first coupling surface 101 perpendicular to the optical axis L is formed at the other side with respect to the optical axis L, and at least one lens is mounted. The optical system part may be variously provided.

For example, the optical system part includes: at least one lens 271 and 272 guiding light along the optical axis L; an optical fiber coupling part 290 disposed opposite to the light source with respect to the lens 271 and 272 and coupled to the optical fiber; a lower optical system holder 300 on which the lens 271 and 272 and the optical fiber coupling part 290 are seated so that the light source, the lens 271 and 272, and the optical fiber coupling part 290 are sequentially arranged to form the optical axis L; and an upper optical system holder 400 disposed above the lower optical system holder 300 and coupled to the lower optical system holder 300 so that the lens 271 and 272 and the optical fiber coupling part 290 are fixedly arranged along the optical axis L.

Here, the first coupling surface 101 is provided on at least one of the lower optical system holder 300 and the upper optical system holder 400, and the light source part 500 is coupled to at least one of the lower optical system holder 300 and the upper optical system holder 400 to slide in a direction perpendicular to the optical axis L in a state in which the second coupling surface 102 closely contacts the first coupling surface 101.

In particular, the first coupling surface 101 is preferably provided on all of the lower optical system holder 300 and the upper optical system holder 400, and the second coupling surface 102 is preferably supported by all of the first coupling surfaces 101 of the lower optical system holder 300 and the upper optical system holder 400 to that the second coupling surface 102 is stably supported by the lower optical system holder 300 and the upper optical system holder 400.

As a component guiding light along the optical axis L, the at least one lens 271 and 272 may be provided in plurality according to the designed optical system.

For example, the at least one lens 271 and 272 may be provided in pair, and the one pair of lenses may be opposite to each other with respect to the optical axis L provided on the upper optical system holder 400 and the lower optical system holder 300, which will be described later.

The one pair of lenses 271 and 272 are spaced apart from each other to provide one optical axis L and constitute the optical system for collecting light in the direction of the optical axis L by using the optical fiber transmitting the light generated from the light source such as the LED device 281 to the optical fiber coupled to the optical fiber coupling part 290. The one pair of lenses 271 and 272 may include a plurality of lenses according to the configuration of the optical system.

As a component installed opposite to the light source such as the LED board 282 with respect to the lenses 271 and 272, the optical fiber coupling part 290 may be variously provided according to the coupling structure of the optical fiber.

For example, the optical fiber coupling part 290 may be a block in which a through-hole 291, to which an adapter (not shown) coupled to an end of the optical fiber is coupled, is defined to transmit light therethrough. The optical fiber coupling part 290 may be coupled to the lower optical system holder 300 and the upper optical system holder 400 by a bolt or the like to fix the lens 271 to the lower optical system holder 300 and the upper optical system holder 400.

Here, the optical fiber coupling part 290 may have various coupling structures. For example, the optical fiber coupling part 290 may be directly coupled or coupled by a separate adaptor.

Also, the optical fiber coupling part 290 may be used as a member for fixing the lens 271 to the lower optical system holder 300 and the upper optical system holder 400, which will be described later, instead of performing a function of the optical fiber coupling part 290.

As the light source such as the LED board 282, the lenses 271 and 272, and the optical fiber coupling part are sequentially arranged to form the optical axis L, the light generated from the light source may be collected to the optical fiber coupled to the optical fiber coupling part 290 through guidance of the lenses 271 and 272.

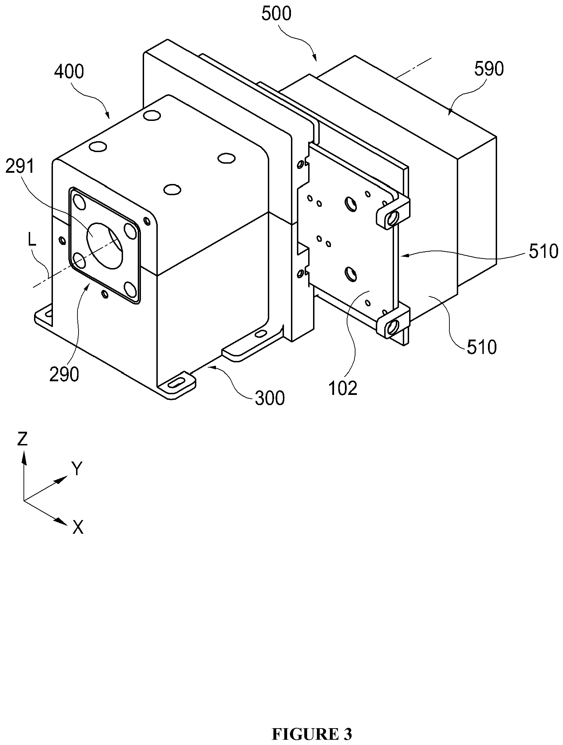

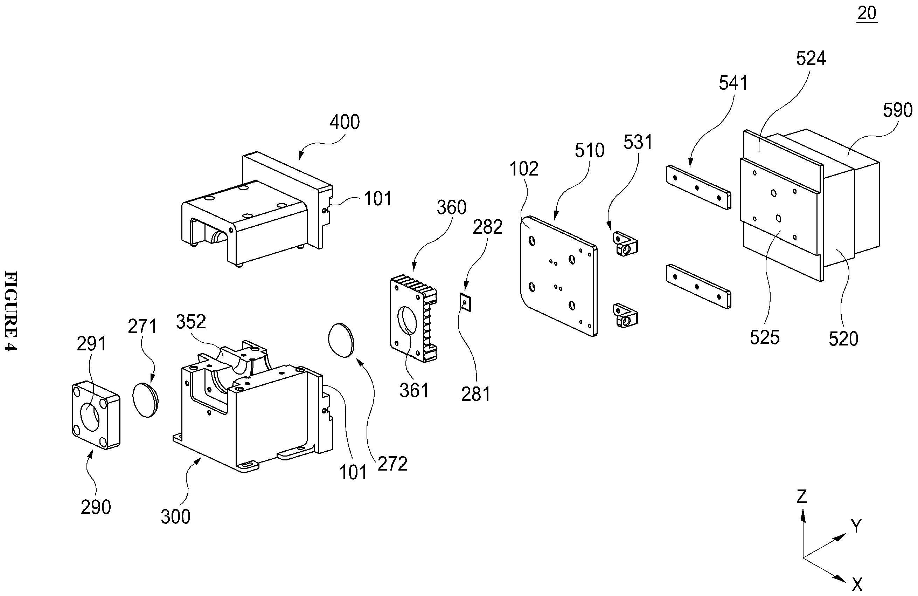

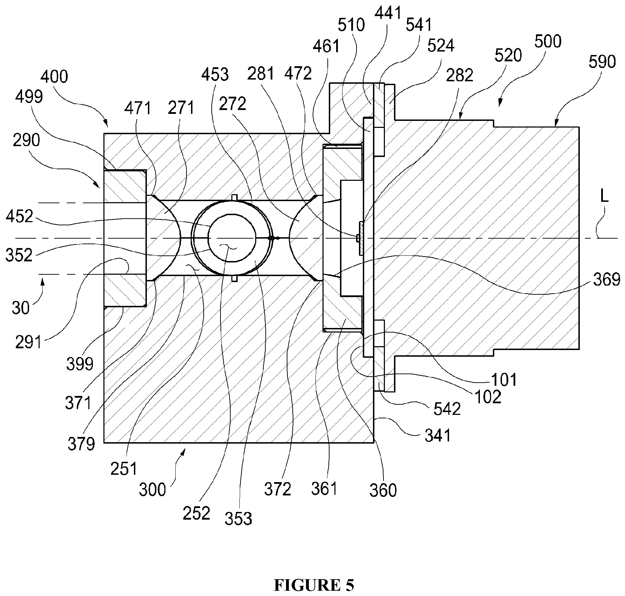

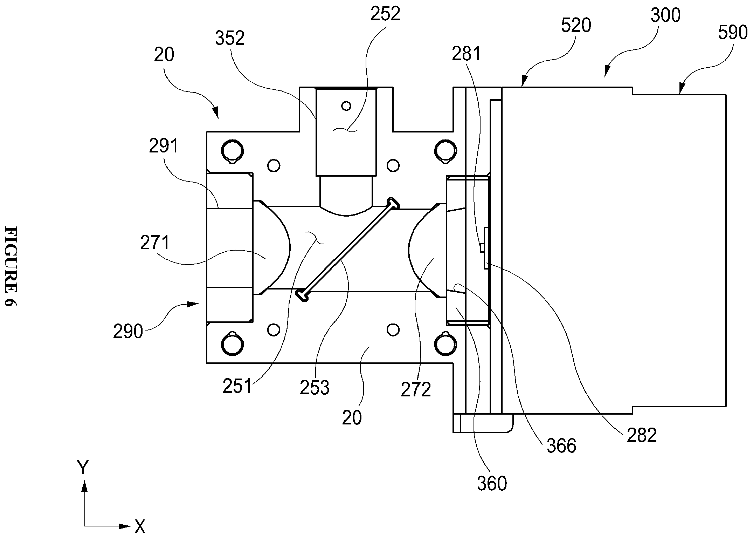

Accordingly, as illustrated in FIGS. 1 to 6, the light source module 20 in accordance with the present invention include: the lower optical system holder 300 on which the lenses 271 and 272 and the optical fiber coupling part 290 are seated so that the light source, the lenses 271 and 272, and the optical fiber coupling part 290 are sequentially arranged to form the optical axis L; and the upper optical system holder 400 disposed above the lower optical system holder 300 and coupled to the lower optical system holder 300 so that the lenses 271 and 272 and the optical fiber coupling part 290 are fixedly arranged along the optical axis L.

As a component on which the lenses 271 and 272 and the optical fiber coupling part 290 are seated so that the light source, the lenses 271 and 272, and the optical fiber coupling part 290 are sequentially arranged to form the optical axis L, the lower optical system holder 300 may be made of various materials such as a synthetic resin material or a metallic material.

In particular, the lower optical system holder 300 forms a three-dimensional structure such as a cylinder and a rectangular parallelepiped when coupled with the upper optical system holder 400, and serve as a jig disposed therebelow when the three-dimensional structure such as a cylinder and a rectangular parallelepiped is vertically divided into upper and lower portions so that the optical axis L passes therebetween.

Also, the lower optical system holder 300 includes a through-hole 251 defined along the optical axis L when coupled with the upper optical system holder 400.

Also, the lower optical system holder 300 includes an insertion groove 399,to which the optical fiber coupling part 290 is inserted, defined in a portion in front of the through-hole 251 so that the light source, the lenses 271 and 272, and the optical fiber coupling part 290 are sequentially arranged to form the optical axis L.

Also, the lower optical system holder 300 includes lens seated parts 371 and 372 on which the lenses 271 and 272 are seated.

Also, the lower optical system holder 300 includes a block seated groove 361, in which a fixing block 360 for fixing the lens 272 is installed, defined in a rear portion that is opposite to the optical fiber coupling part 290, i.e., a portion at which the light source is disposed.

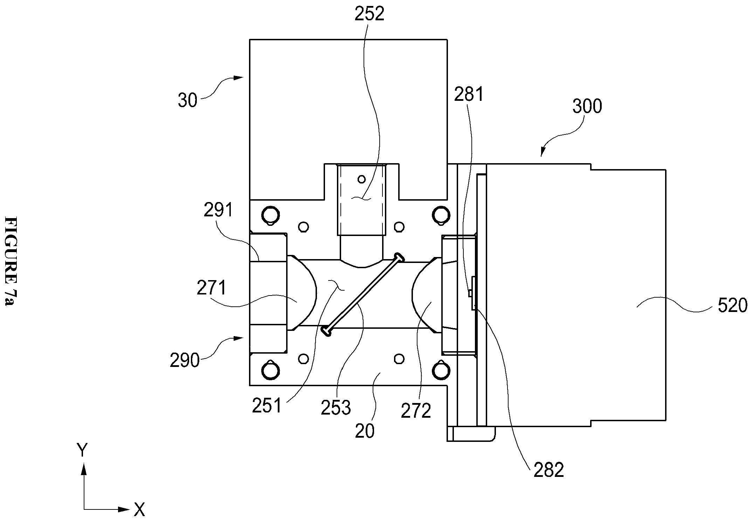

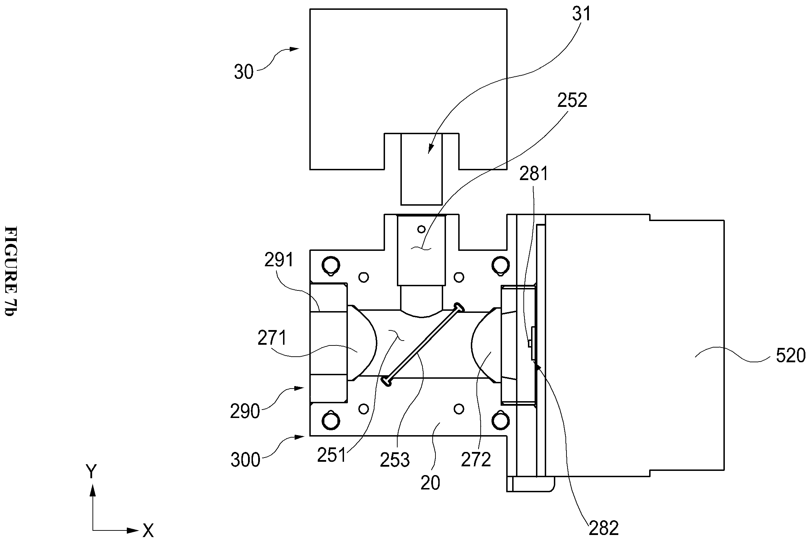

Also, as illustrated in FIGS. 7A and 7B, the lower optical system holder 300 includes a member insertion groove 353, in which at least one light path conversion member 253 is installed to reflect a direction of light generated from an auxiliary light source module 30 into the direction of the optical axis L when the auxiliary light source module 30, which will be described later, is coupled in a direction perpendicular to the optical axis L.

Here, in the lower optical system holder 300, a portion 352 of an auxiliary through-hole 252 is defined in a direction perpendicular to the direction of the optical axis L so that the auxiliary light source module 30 is coupled in the direction perpendicular to the optical axis L.

Also, in the lower optical system holder 300, one of a protruding portion or a groove portion may be defined for precise coupling with the upper optical system holder 400.

Also, a guide block (not shown) may be coupled to a bottom surface of the lower optical system holder 300 so that at least one guide member (not shown) installed in the housing is guided.

The upper optical system holder 400 may be made of various materials such as a synthetic resin material or a metallic material so that the light source, the lenses 271 and 272, and the optical fiber coupling part 290 are fixedly arranged along the optical axis L.

In particular, the upper optical system holder 400 forms a rectangular parallelepiped when coupled with the lower optical system holder 300 and serve as a jig disposed thereabove when the rectangular parallelepiped is vertically divided into upper and lower portions so that the optical axis L passes therebetween.

Also, the upper optical system holder 400 includes a through-hole 251 defined along the optical axis L while being coupled with the lower optical system holder 300.

Also, the upper optical system holder 400 includes an insertion groove 499, to which the optical fiber coupling part 290 is inserted, defined in front of the through-hole 251 so that the light source, the lenses 271 and 272, and the optical fiber coupling part 290 are sequentially arranged to form the optical axis L.

Also, the upper optical system holder 400 includes lens seated parts 471 and 472 on which the lenses 271 and 272 are seated.

Also, the upper optical system holder 400 includes a block seated groove 461, in which a fixing block 360 for fixing the lens 272 is installed, defined in a rear portion that is opposite to the optical fiber coupling part 290, i.e., a portion at which the light source is disposed

As a component for fixing the lens 272 to the lower optical system holder 300 and the upper optical system holder 400, the fixing block 260 includes a through-hole 361 so that light generated from the LED device 281 is transmitted therethrough.

Also, the fixing block 360 may be preferably made of a metallic material to dissipate heat transmitted from the LED device 281 through thermal radiation, and may include a plurality of projections and ribs on an outer circumferential surface to maximize a heat dissipation effect.

As described above, the lens seated parts 371, 372, 471, and 472, which are defined by the lower optical system holder 300 and the upper optical system holder 400, may stably fix the lenses 271 and 272 and serve to couple the lower optical system holder 300 and the upper optical system holder 400 without distortion.

Also, the lens seated parts 371, 372, 471, and 472, which are defined by the lower optical system holder 300 and the upper optical system holder 400, may allow the lenses to be positioned at exact positions.

As illustrated in FIGS. 7A and 7B, the upper optical system holder 400 includes a member insertion groove 453, in which a light path conversion member 253 is installed to reflect a direction of light generated from an auxiliary light source module 30 into the direction of the optical axis L when the auxiliary light source module 30, which will be described later, is coupled in a direction perpendicular to the optical axis L.

Here, in the upper optical system holder 400, a portion of an auxiliary through-hole 252 is defined in a direction perpendicular to the direction of the optical axis L so that the auxiliary light source module 30 is coupled in the direction perpendicular to the optical axis L.

As described above, for exact coupling between the upper optical system holder 400 and the lower optical system holder 300, a plurality of projections may be defined in one, and a plurality of grooves, to which the plurality of projections are inserted, may be defined in the other. Thus, the upper optical system holder 400 and the lower optical system holder 300 may be precisely coupled to each other.

The light source module includes a linear guide part that guides slide movement of the light source part 500 with respect to the optical system part 30 in the direction perpendicular to the optical axis L in a state in which the second coupling surface 102 closely contacts the first coupling surface 101.

For example, as a component that guides slide movement of the light source part 500 with respect to the optical system part 30 in the direction perpendicular to the optical axis L in a state in which the second coupling surface 102 closely contacts the first coupling surface 101, the linear guide part may be variously provided.

For example, as a component that is coupled to at least one of the lower optical system holder 300 and the upper optical system holder 400 and guides the linear slide movement of the metal plate 510 in the direction perpendicular to the optical axis L while the second coupling surface 102 of the metal plate 510 closely contacts the first coupling surface 101, the linear guide part may be variously provided.

In particular, the linear guide part may include one pair of slide members 541.

The one pair of slide members 541 may be a plate member that is elongated along the slide movement direction to guide the linear slide movement of the metal plate 510 in the direction perpendicular to the optical axis L while allowing the second coupling surface 102 of the metal plate 510 to closely contact the first coupling surface 101.

Also, portions 341 and 441 to which the slide members 541 are coupled may further protrude toward the light source part 500 to prevent interference with the metal plate 510 when the lower optical system holder 300 and the upper optical system holder 400, to which the one pair of slide members 541 are coupled, move in a slide manner.

Also, at least one of the metal plate 510 and the heat dissipation block 520 may be additionally coupled to at least one stopper 531, which restricts movement of the light source part 500 with respect to the lower optical system holder 300 and the upper optical system holder 400 so that the LED device 281 coupled to the metal plate 510 is exactly positioned to the optical axis L.

The stopper 532 may restrict the movement of the light source part 500 with respect to the lower optical system holder 300 and the upper optical system holder 400 and allow the LED device 281 to be exactly positioned to the optical axis L by a user.

As illustrated in FIGS. 7A and 7B, the light source module 20 having the above-described configuration may be additionally coupled to at least one auxiliary light source module 30 that is coupled in the direction perpendicular to the optical axis L to transmit light in combination with the light generated from the LED device 281 or independently transmit auxiliary light to the optical fiber coupled to the optical fiber coupling part 290.

To this end, the light source module 20 includes an auxiliary through-hole 252 define din the direction perpendicular to the direction of the optical axis L so that a light emitting part of the auxiliary light source module 30 is inserted thereto.

As a component coupled in the direction perpendicular to the optical axis L to transmit light combined with the light generated from the LED device 281 or independent auxiliary light through the light emitting part, the auxiliary light source module 30 may be variously provided.

The auxiliary light source module 30 may include various light sources according to purposes such as R, G, B, white light source, and near infrared (NIR).

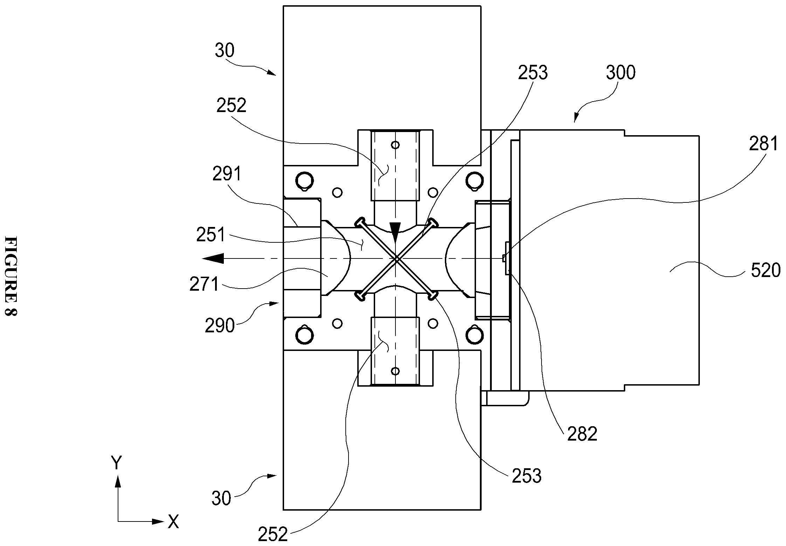

The auxiliary light source module 30 may be provided in plurality with respect to the optical system part. The auxiliary light source module 30 may be variously provided. For example, the plurality of auxiliary light source modules 30 may be disposed opposite to each other as illustrated in FIG. 8 or arranged in order along a longitudinal direction of the optical system part.

Here, the light path conversion member 253 is installed at an appropriate position so that light emitted from each of the auxiliary light source modules 30 is emitted in the direction of the optical axis L.

As a member converting the light emitting from the auxiliary light source module 30 in the direction of the optical axis L, the may be variously provided. For example, the light path conversion member 253 may include a semi-transmission mirror, or a filter reflecting or transmitting light having a predetermined wavelength may be formed or attached to the light path conversion member 253.

Although the above description merely corresponds to some exemplary embodiments that may be implemented by the present disclosure, as well known, the scope of the present disclosure should not be interpreted as being limited to the above-described embodiments, and all technical spirits having the same basis as that of the above-described technical spirit of the present disclosure are included in the scope of the present disclosure.

## Claims

1. A medical light source module comprising:
an optical system part in which an optical fiber is coupled to one side, and a first coupling surface (101), which is perpendicular to an optical axis (L), is provided at the other side with respect to the optical axis (L), and to which at least one lens is mounted to provide an optical system; and
a light source part (500) having a second coupling surface (102), which surface-contacts the first coupling surface (101), and being installed so that an optical axis of a light source is perpendicular to the second coupling surface (102).

2. The medical light source module of claim 1, wherein the light source part (500) comprises:
a metal plate (510) having the second coupling surface;
the light source coupled to the metal plate (510) so that the optical axis is perpendicular to the second coupling surface (102); and
a heat dissipation block (520) coupled to at least one of the metal plate (510) and the light source to dissipate heat generated from the light source.

3. The medical light source module of claim 2, wherein the light source comprises a LED device (281) and a LED board (282) on which the LED device (281) is installed and installed on the second coupling surface (102).

4. The medical light source module of claim 2, wherein the optical system part comprises:
at least one lens (271, 272) configured to guide light along the optical axis (L);
an optical fiber coupling part (290) installed opposite to the light source with respect to the lens (271, 272) and coupled to an optical fiber;
a lower optical system holder (300) on which the lens (271, 272) and the optical fiber coupling part (290) are seated so that the light source, the lens (271, 272), and the optical fiber coupling part (290) are sequentially arranged to provide the optical axis (L);
an upper optical system holder (400) disposed above the lower optical system holder (300) and coupled to the lower optical system holder (300) so that the lens (271, 272) and the optical fiber coupling part (290) are fixedly arranged along the optical axis (L),
wherein the first coupling surface (101) is provided on at least one of the lower optical system holder (300) and the upper optical system holder (400), and
the light source part (500) is coupled to at least one of the lower optical system holder (300) and the upper optical system holder (400) to slide in a direction perpendicular to the optical axis (L) in a state in which the second coupling surface (102) closely contacts the first coupling surface (101).

5. The medical light source module of claim 4, wherein the optical system part further comprises a fixing block (360) which is disposed opposite to the optical fiber coupling part (290) with respect to the lens (271, 272) to fix the lens (272) with respect to the lower optical system holder (300) and the upper optical system holder (400) and in which a through-hole (361) is defined to transmit light generated from the light source.

6. The medical light source module of claim 5, wherein the fixing block (360) is made of a metallic material to discharge heat transferred from the light source, and at least one of a plurality of projections and ribs are formed on an outer circumferential surface to maximize a heat dissipation effect.

7. The medical light source module of claim 4, further comprising a linear guide part configured to guide a slide movement of the light source part (500) with respect to the optical system part (30) in a direction perpendicular to the optical axis (L) in a state in which the second coupling surface (102) closely contacts the first coupling surface (101).

8. The medical light source module of claim 7, wherein the linear guide part comprises one pair of slide members (541) coupled to at least one of the lower optical system holder (300) and the upper optical system holder (400), and configured to guide a linear slide movement of the metal plate (510) in a direction perpendicular to the optical axis (L) while the second coupling surface (102) of the metal plate (510) closely contacts the first coupling surface (101).

9. The medical light source module of claim 8, wherein a block coupling surface (525) of the heat dissipation block (520), which is perpendicular to the optical axis (L), further protrudes in a front direction than the rest portion (524) so that the metal plate (510) is coupled to the heat dissipation block (520) while being spaced apart from both sides of the heat dissipation block (520).

10. The medical light source module of claim 8, wherein each of the one pair of slide members (541) is a plate member that is elongated along the slide movement direction, and
each of portions (341, 441) coupled with the slide member (541) further protrudes toward the heat dissipation block (520) to prevent interference with the metal plate (510) when the lower optical system holder (300) and the upper optical system holder (400), which are coupled with the one pair of slide members (541), move in a slide manner.

11. The medical light source module of claim 3, wherein at least one of the metal plate (510) and the heat dissipation block (520) is additionally coupled to at least one stopper (531) that restricts a movement of the light source part (500) with respect to the lower optical system holder (300) and the upper optical system holder (400) so that the LED device (281) coupled to the metal plate (510) is exactly positioned on the optical axis (L).

12. A medical light source device comprising:
a housing (10) installed to expose an operation panel (11), which is operated by a user, to the outside; and
a light source module (20) installed to expose an optical fiber coupling part (290), which is disposed at one side of the housing (10) and coupled with an optical fiber, to the outside of the housing (10),
wherein the light source module (20) is attached to and detached from the housing (10) in a sliding manner in a direction perpendicular to the optical axis (L), and is the same as the light source module (20) in accordance with claims 1 to 11.

13. The medical light source device of claim 12, further comprising at least one auxiliary light source module (30) that is additionally coupled in the direction perpendicular to the optical axis (L) to transmit light in combination with light generated from the LED device (281) or independently transmit auxiliary light to an optical fiber coupled to the optical fiber coupling part (290).
